# EUROPEAN PATENT APPLICATION

(11) **EP 4 009 043 A1**
(43) Date of publication of application: **08.06.2022**
(21) Application number: 21209404.9
(22) Date of filing: 19.11.2021
(51) Int. Cl.: G01N 27/12, G01N 27/404

(54) **GAS DETECTION DEVICE FOR GASEOUS COMPOUND**

(30) Priority: 01.12.2020 US 202063119791 P
(71) Applicant: Carrier Corporation, Palm Beach Gardens, FL 33418 (US)
(72) Inventor: RANJAN, Rajiv, Bloomfield, 06002 (US); SILVER, Travis, Colorado Springs, 80919 (US); BAILEY, Callum, Bradenton, 34202 (US)
(74) Representative: Dehns

(57) **Abstract**

Disclosed herein is a gas detection device (10) comprising a membrane filter (100) disposed between an electrochemical sensor (12) and an environment exterior to the gas detection device (10).

## Description

The present invention relates to a gas detection device and to a related method of protecting a sensor. Exemplary embodiments pertain to the art of electrochemical sensors for gaseous compounds.

Many electrochemical sensors utilize a membrane electrode assembly (MEA) or semiconductor metal oxide to detect specific compounds. The lifetime and performance of electrochemical sensors for gaseous compounds may be limited by the durability of the electrochemical sensor. The durability of the electrochemical sensor can be impacted by exposure to chemical compounds that poison the MEA or the semiconductor metal oxide. There is therefore a need for an improved gas detection device that protects the electrochemical sensor from chemical compounds that poison the MEA or semiconductor metal oxide.

According to a first aspect of the invention a gas detection device is provided, the device comprising a membrane filter disposed between an electrochemical sensor or a semiconductor metal oxide and an environment exterior to the gas detection device.

The electrochemical sensor may comprise a membrane electrode assembly.

The membrane filter may comprise a molecular sieve, a polymer, or a combination thereof.

The molecular sieve may comprise zeolites, metal organic frameworks, engineered activated carbon, clay, and combinations thereof.

The polymer may have a selectivity greater than or equal to 25:1 for a target compound and the gas detection device may be capable of detecting the target compound.

The membrane filter may comprise a combination of a molecular sieve and a polymer.

The membrane filter may have a thickness of 10 to 5000 micrometers.

According to a second aspect of the invention a method of protecting a sensor is provided, the method including disposing a membrane filter between the sensor and an exterior environment wherein the sensor comprises an electrochemical sensor or a semiconductor metal oxide.

The electrochemical sensor may include a membrane electrode assembly or a semiconductor metal oxide.

The membrane filter may include a molecular sieve, a polymer, or a combination thereof.

The molecular sieve may comprise zeolites, metal organic frameworks, activated carbon, clay, and combinations thereof.

The polymer may have a selectivity greater than or equal to 25:1 for a target compound and the sensor may be capable of detecting the target compound.

The membrane filter may comprise a combination of a molecular sieve and a polymer.

The membrane filter may have a thickness of 10 to 5000 micrometers.

According to a third aspect of the invention a method of detecting a gaseous compound in an environment is provided, the method comprising exposing a gas detection device to the environment wherein the gas detection device comprises an electrochemical sensor or a semiconductor metal oxide sensor and a membrane filter disposed between the electrochemical sensor or the semiconductor metal oxide sensor and the environment; and detecting the gaseous compound with the sensor.

The electrochemical sensor may comprise a membrane electrode assembly.

The membrane filter may comprise a molecular sieve, a polymer, or a combination thereof.

The molecular sieve may comprise zeolites, metal organic frameworks, activated carbon, clay, and combinations thereof.

The polymer may have a selectivity greater than or equal to 25:1 for a target compound and the gas detection device may be capable of detecting the target compound.

The membrane filter may have a thickness of 10 to 5000 micrometers.

Certain exemplary embodiments will now be described in greater detail by way of example only and with references to the accompanying drawings in which:
FIG. 1 is a schematic diagram of a gas detection device;
FIG. 2 is a schematic diagram of an electrochemical sensor with an MEA;
FIG. 3 is an exploded view of an electrochemical sensor with an MEA; and
FIG. 4 is schematic diagram of a sensor with a semiconductor metal oxide.

A detailed description of one or more embodiments of the disclosed apparatus and method are presented herein by way of exemplification and not limitation with reference to the Figures.

Many sensors for gaseous compounds use an adsorbent such as carbon to protect the sensor from compounds that would poison the membrane electrode assembly (MEA) or semiconductor metal oxide. Adsorbents offer limited protection as the adsorbent has a finite capacity to remove/contain chemical compounds. Once that capacity is reached the adsorbent must be regenerated and/or replaced. Additionally, adsorbents are frequently used in sufficient quantity that the resulting device is fairly bulky which can result in limitations on the placement of the sensing device.

In contrast a membrane filter offers several advantages such as longer protection and a device that is smaller in size. The filter blocks chemical compounds that would poison the sensor but does not bind or sequester them as adsorbents do. As a result, the membrane filter has greater longevity than an adsorbent resulting in a longer lived, more robust device. Furthermore, adsorbents can have varying selectivity but using a membrane filter allows a targeted exclusion of specified compounds based on size.

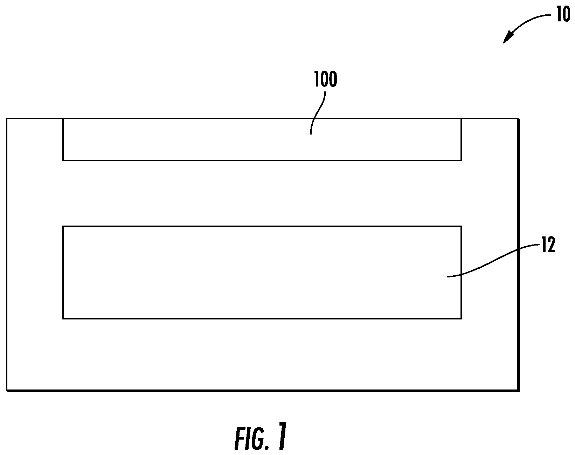

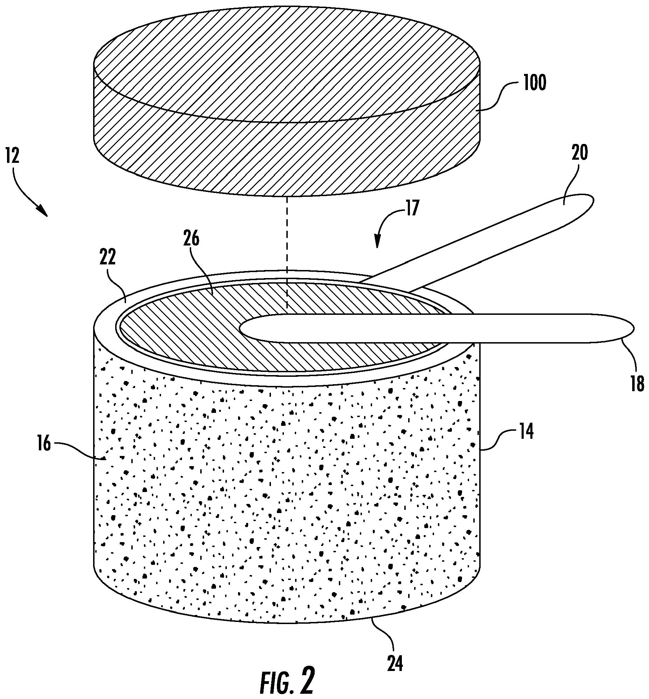

FIG. 1 schematically illustrates a gas detection device, the gas detection device generally indicated at 10. The gas detection device 10 may be configured to detect carbon monoxide, volatile organic chemicals (VOCs), explosive gasses such as ethane, propane, methane, hydrogen sulfide (H₂S), oxygen and hydrogen. The gas detection device 10 includes a sensor 12 disposed therein, wherein the sensor includes an electrochemical sensor. Gas detection device 10 further includes membrane filter 100. Membrane filter 100 forms a protective interface between the electrochemical sensor and the environment outside the gas detection device. As shown in FIG. 2, the sensor 12 may include a vessel 14 configured to hold an aqueous solution 16. The vessel 14 may be formed from a plastic or a conductive metal. The aqueous solution 16 may be water, a concentrated salt solution (such as a concentrated salt solution of sodium chloride or lithium chloride) or an acid aqueous solution (such as a sulfuric acid solution).

As shown in FIG. 2, the sensor 12 further includes a sensing element 17 operably coupled to the vessel 14. The sensing element includes a first electrode 18, a second electrode 20 and a membrane electrode assembly 26.

The membrane electrode assembly 26 is disposed between the first electrode 18 and the second electrode 20. The second electrode 20 is operably coupled to a top outer surface 22 of the vessel 14 and located adjacent to the aqueous solution 16. In embodiments where the vessel 14 is made from a conductive metal, the second electrode 20 may be operably coupled to any outer surface, for example the bottom outer surface 24, of the vessel 14. When the membrane electrode assembly 26 becomes hydrated from the aqueous solution 16, ions become highly mobile. The membrane electrode assembly 26 is an assembled stack of polymer electrolyte membrane (PEM) or alkali anion exchange membrane (AAEM) that allows the transport of the protons or hydroxide ions from the first electrode 18 to the second electrode 20 through the membrane electrode assembly 26 but forces the electrons to travel around a conductive path to the first electrode 18. The membrane electrode assembly 26 is not in direct contact with the aqueous solution in order to prevent flooding or degradation of the assembly 26. It will also be appreciated that the first electrode 18 and the second electrode 20 are not in direct contact with the aqueous solution to prevent corrosion of the first electrode 18 and the second electrode 20. The membrane electrode assembly 26 is at least partially protected from chemical compounds that would poison the membrane electrode assembly by a membrane filter 100.

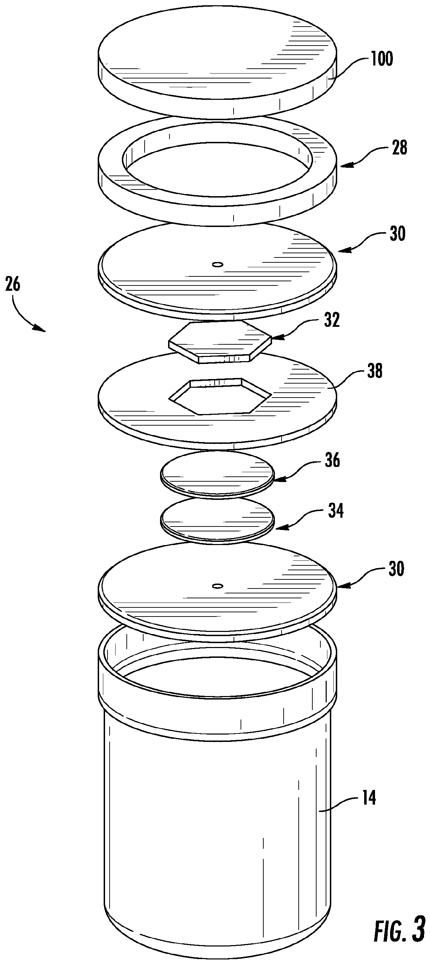

As shown in FIG. 3, the membrane electrode assembly 26 may further include a first gasket 28 and two disks with flow channel 30, a first gas diffusion layer 32 and a second gas diffusion layer 34. The first gas diffusion layer 32 and the second gas diffusion layer 34 are configured to ensure proper transport of gases, electrons, and heat of reaction. It will be appreciated that the first gas diffusion layer 32 and the second gas diffusion layer 34 may be a carbon paper (e.g., Toray paper to name one non-limiting example).

The membrane electrode assembly 26 may also further include an ion exchange membrane 36 disposed adjacent to the second gas diffusion layer 34. The ion exchange member includes a catalyst disposed thereon. The catalyst layers are typically composed of noble-metal catalyst, such as platinum or platinum-alloys, supported on carbon with an ionomer binder, which is the same polymeric material as the ion-exchange membrane. The ion exchange membrane promotes the transport of ions between a first catalyst layer and a second catalyst layer. The membrane electrode assembly 26 further includes a washer 38 disposed between the ion exchange membrane 36 and the first gas diffusion layer 32. It will be appreciated that the washer 38 is configured to separate the first gas diffusion layer 32 and the second gas diffusion layer 34 in order to reduce the likelihood of short-circuiting the cell since both are electrically conductive, and provides a solid surface for the gasket 28 to sit and form an airtight seal. The membrane assembly 26 is at least partially protected from chemical compounds that would poison the membrane electrode assembly by a membrane filter 100.

The membrane electrode assembly 26 may include a first catalyst layer (not shown) and a second catalyst layer (not shown) disposed adjacent to the first gas diffusion layer 32 and the second gas diffusion layer 34, respectively. The first gas diffusion layer 32 and the second gas diffusion layer 34 are further configured to distribute the reactants uniformly along the active surface of the first catalyst layer and the second catalyst layer from gas flow channels that are part of disks 30.

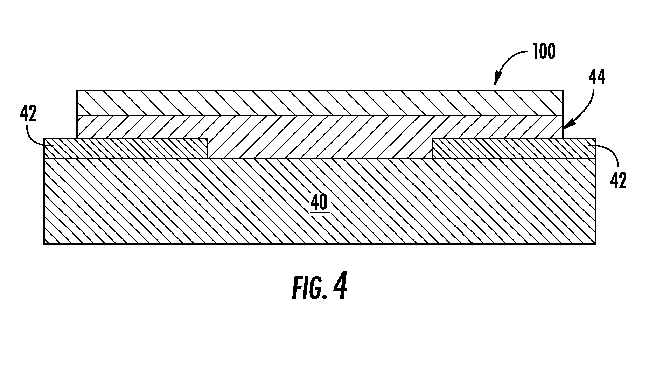

FIG. 4 schematically illustrates a semiconductor metal oxide type gas detection device. The device includes a substrate 40 with electrodes 42 disposed on the substrate 40. Gas sensing layer 44 is disposed between the electrodes 42. Membrane filter 100 is disposed between the gas sensing layer 44 and the exterior environment.

The membrane filter 100 may be molecular sieve based, polymer based or may be a hybrid filter comprising both molecular sieves and polymer. The membrane filter 100 prevents some or all of the chemical compounds that would poison the membrane electrode assembly from entering the sensing element. Exemplary molecular sieves include zeolites, metal organic frameworks, engineered activated carbon, clay, and combinations thereof. Engineered activated carbon is designed and produced to have a specific pore size and thus differs from an adsorbent activated carbon. The pore size of the molecular sieves is chosen based on the size of the molecules that are allowed to pass through as well as the size of the molecules that are to be excluded. For example, the pore size may be chosen to be big enough to allow carbon monoxide through but small enough to exclude compounds such as styrene, cyclohexanone and other cyclic compounds. Exemplary pore sizes include 2 to 10 micrometers.

Exemplary polymers include polyamide and low-density polyethylene. The polymer is chosen based on its selectivity for the target chemical compound (the chemical compound being detected). In some embodiments the polymer has a selectivity greater than or equal to 25:1, or greater than or equal to 50:1, or greater than or equal to 100:1 for the target chemical compound.

A hybrid filter may take one of several forms such as a molecular sieve deposited on a polymer layer, a molecular sieve dispersed in a polymer - either randomly or in layers, or a combination thereof.

The membrane filter may have a thickness greater than or equal to 10 micrometers and less than or equal to 5000 micrometers. Within this range the selective membrane may have a thickness greater than or equal to 20 micrometers, or, greater than or equal to 50 micrometers. Also, within this range, the selective membrane may have a thickness less than or equal to 1000 micrometers, or, less than or equal to 500 micrometers.

The membrane filter offers several advantages compared to an adsorbent. Adsorbents have a limited lifetime. Additionally, adsorbents may not provide adequate protection to the sensor due to an inability to adsorb all of the chemical compounds that can poison the membrane electrode assembly. Adsorbents can also decrease sensor sensitivity by removing a portion of the compound to be detected (the target compound) as adsorbents frequently are not selective. Adsorbents may also allow the aqueous solution 16 to be depleted over time by evaporation. When using an electrochemical sensor having an aqueous solution, using the membrane filter reduces evaporation which extends the life of the sensor. Stated another way a sensor having a membrane filter disposed between the sensor and the exterior environment has a lower aqueous solution evaporation rate than a sensor having an adsorbent layer disposed between the sensor and the exterior environment.

The terminology used herein is for the purpose of describing particular embodiments only and is not intended to be limiting of the present disclosure. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, element components, and/or groups thereof. Furthermore, the terms "comprises" and/or "comprising," as well as the terms "includes" and/or "including," support embodiments which do not incorporate elements other than those described.

While the present invention has been described with reference to an exemplary embodiment or embodiments, it will be understood by those skilled in the art that various changes may be made and equivalents may be substituted for elements thereof without departing from the scope of the present invention as defined by the claims. In addition, many modifications may be made to adapt a particular situation or material to the teachings of the present invention without departing from the scope of the invention as set out in the appended claims. Therefore, it is intended that the present invention not be limited to the particular embodiment disclosed as the best mode contemplated for carrying out this present invention, but that the present invention will include all embodiments falling within the scope of the claims.

## Claims

1. A gas detection device (10) comprising a membrane filter (100) disposed between an electrochemical sensor (12) or a semiconductor metal oxide and an environment exterior to the gas detection device (10).

2. The gas detection device (10) of claim 1, wherein the electrochemical sensor (12) comprises a membrane electrode assembly (26).

3. The gas detection device (10) of claim 1 or 2, wherein the gas detection device (10) has an evaporation rate less than a gas detection device (10) having an adsorbent disposed between an electrochemical sensor (12) and an environment exterior to the gas detection device (10).

4. The gas detection device (10) of claim 1, 2 or 3, wherein the membrane filter (100) comprises a molecular sieve, a polymer, or a combination thereof.

5. The gas detection device (10) of claim 4, wherein the molecular sieve comprises zeolites, metal organic frameworks, engineered activated carbon, clay, and combinations thereof.

6. The gas detection device (10) of claim 4, wherein the polymer has a selectivity greater than or equal to 25:1 for a target compound and the gas detection device (10) is capable of detecting the target compound.

7. The gas detection device (10) of claim 4, wherein the membrane filter (100) comprises a combination of a molecular sieve and a polymer.

8. The gas detection device (10) of any preceding claims, wherein the membrane filter (100) has a thickness of 10 to 5000 micrometers.

9. A method of protecting a sensor (12) comprising disposing a membrane filter (100) between the sensor (12) and an exterior environment wherein the sensor (12) comprises an electrochemical sensor (12) or a semiconductor metal oxide.

10. The method of claim 9, wherein the electrochemical sensor (12) comprises a membrane electrode assembly (26).

11. The method of claim 9 or 10, wherein the membrane filter (100) comprises a molecular sieve, a polymer, or a combination thereof.

12. The method of claim 11, wherein the molecular sieve comprises zeolites, metal organic frameworks, engineered activated carbon, clay, and combinations thereof.

13. The method of claim 11, wherein the polymer has a selectivity greater than or equal to 25:1 for a target compound and the sensor (12) is capable of detecting the target compound.

14. The method of any of claims 9 to 13, wherein the membrane filter (100) has a thickness of 10 to 5000 micrometers.

15. A method of detecting a gaseous compound in an environment comprising the gas detection device (10) of any of claims 1 to 8.
